# EUROPEAN PATENT APPLICATION

(11) **EP 4 748 406 A1**
(43) Date of publication of application: **27.05.2026**
(21) Application number: 24843160.3
(22) Date of filing: 18.07.2024
(51) Int. Cl.: A61L 15/26, A61K 9/70, A61K 47/14, A61K 47/34, C09J 7/38, C09J 11/06, C09J 133/14

(54) **MEDICAL ADHESIVE AGENT AND MEDICAL ADHESIVE SHEET**

(30) Priority: 19.07.2023 JP 2023117593
(71) Applicant: Nitto Denko Corporation, Ibaraki-shi, Osaka 567-8680 (JP)
(72) Inventor: MURATA, Tatsuya, Ibaraki-shi, Osaka 567-8680 (JP); YAEGASHI, Yuko, Ibaraki-shi, Osaka 567-8680 (JP); HIRANO, Keisuke, Ibaraki-shi, Osaka 567-8680 (JP)
(74) Representative: Grünecker Patent- und Rechtsanwälte PartG mbB
(86) International application number: PCT/JP2024/025718
(87) International publication number: WO 2025/018371

(57) **Abstract**

A medical pressure-sensitive adhesive (PSA) is provided. The PSA contains a water-dispersed copolymer obtained by copolymerizing a monomer mixture containing an alkyl (meth)acrylate, a carboxy group-containing monomer, an N-containing monomer, and a silane-based monomer, and an organic liquid component having more than one hydroxy group in a molecule.

## Description

### [Technical Field]

The present invention relates to a medical pressure-sensitive adhesive (PSA) and a medical pressure-sensitive adhesive sheet.

The application claims the priority based on Japanese Patent Application No. 2023-117593, filed on July 19, 2023, the content of which is herein incorporated by reference in its entirety.

### [Background Art]

Various medical PSAs and medical PSA sheets prepared with the PSAs has been widely used for the purposes of protection of an affected area on skin, transdermal absorption of a drug, and fixation of gauze, a tube, or the like to skin. Such medical PSAs and medical PSA sheets are used in the forms of, e.g., adhesive bandages, surgical tapes, first-aid adhesive tapes, large adhesive tapes, dressing materials, and cataplasm materials. Examples of related technical literatures disclosing this kinds of conventional arts include Patent Documents 1 to 2. Patent Documents 1 to 2 disclose PSAs used for attachment to skin in medical application.

### [Citation List]

### [Patent Literature]

[Patent Document 1] Japanese Patent Application Publication No. 2002-315775
[Patent Document 2] Japanese Patent Application Publication No. 2008-113763

### [Summary of Invention]

### [Technical Problem]

PSAs attached to skin in medical applications are required to have good tight adhesiveness to skin, and also desired to cause no damage such as exfoliation of keratin layers during peeling from skin and to enable detachment with less irritation to skin and no pain. It is desirable to provide no transfer or residual of a PSA component on skin after peeling of a PSA and to prevent pollution of the skin by the PSA component. For example, Patent Document 1 describes addition of 60 parts by weight of a specific liquid carboxylate relative to 100 parts by weight of an acrylic copolymer in an acrylic PSA leads to reduction in skin irritancy. Patent Document 2 also discloses investigation of a composition to which 30 parts by weight of sorbitan trioleate, sorbitan monolaurate, or sorbitan monooleate relative to 100 parts by weight of an acrylic copolymer were added in an acrylic PSA.

In addition, a PSA attached to skin has a risk of having moisture generated on an adherent interface of the PSA by sweating of the skin, which is an adherend, thereby reducing tight adhesive force and causing peeling, and thus is required to have water resistance that can maintain tight adhesiveness to skin even in sweating.

Meanwhile, in recent years, there has also been a trend toward independence from and reduction of an organic solvent in PSAs, in view of environmental friendliness, health of an operator, and other factors. Turn to the related technical literatures, PSAs prepared with a water-dispersed copolymer as disclosed in Patent Document 2 are more desirable than PSAs prepared with an organic solvent, such as toluene, disclosed in Patent Document 1. However, PSAs with a water-dispersed copolymer typically tend to have lower water resistance than solvent-based PSAs prepared with an organic solvent, and thus is disadvantageous in, e.g., application for attachment to skin, which may sweat as described above. The inventors investigated and revealed that the PSA described in Patent Document 2 also have room for improvement in water resistance.

The inventors further investigated earnestly, and consequently successfully created a PSA that has improved water resistance as well as retains performance for providing less skin irritation in peeling from the skin, and completed the present invention. In other words, the present invention has an objective to provide a PSA that causes less irritation to skin in peeling from the skin, good water resistance, and an excellent capability of preventing pollution of skin. Another related objective is to provide a PSA sheet containing the PSA.

### [Solution to Problem]

The specification provides a medical PSA. The PSA contains a water-dispersed copolymer obtained by copolymerizing a monomer mixture and an organic liquid component having more than one hydroxy group in a molecule. The monomer mixture contains an alkyl (meth)acrylate, a carboxy group-containing monomer, a nitrogen-atom-containing (N-containing) monomer and a silane-based monomer. With such a composition, the PSA can provide less irritation to skin in peeling from the skin, good water resistance, and further, a capability of preventing pollution of skin by a PSA component.

It should be noted that the PSA described in Patent Document 2 does not have water resistance according to the art disclosed herein, as shown in the results of Comparative Example 4 described later. For example, reduction in the amount of an organic liquid component for the purpose of improving water resistance of such a PSA may diminish performance for reducing skin irritation in peeling. Patent Document 2 does not suggest the combination of prevention of skin irritation in peeling and good water resistance by the art disclosed herein.

In some preferred embodiments, the amount of the N-containing monomer in 100 parts by mass of the monomer mixture is 0.1 to 10 parts by mass; in other words, the content of the N-containing monomer in the monomer mixture is 0.1 to 10% by mass. The use of the N-containing monomer in this range can preferably achieve improved water resistance of a PSA.

In some preferred embodiments, the N-containing monomer has a structure in which a nitrogen atom is bound to no hydrogen atoms. With such a structure, the use of the N-containing monomer can preferably achieve improved water resistance of a PSA.

In some preferred embodiments, the N-containing monomer has a cyclic structure containing a nitrogen atom in a ring. With such a structure, the use of the N-containing monomer is likely to provide more excellent water resistance.

In some preferred embodiments, the amount of the carboxy group-containing monomer in 100 parts by mass of the monomer mixture is 0.1 to 10 parts by mass; in other words, the content of the carboxy group-containing monomer in the monomer mixture is 0.1 to 10% by mass. The use of the carboxy group-containing monomer in this range can preferably provide good water resistance.

In some preferred embodiments, the organic liquid component has a hydrophile-lipophile balance (HLB) of 3 to 16. With a HLB in the range of 3 to 16, the use of the organic liquid component can better combine reduction of skin irritation in peeling and good water resistance. With such a HLB, the organic liquid component is likely to provide excellent prevention of pollution of skin.

In some preferred embodiments, the organic liquid component is a fatty acid ester derivative of polyhydric alcohol. The art disclosed herein can be preferably performed in an embodiment that employs the organic liquid component with this structure. In particular, a substance in which a fatty acid forming the fatty acid ester derivative has 8 to 18 carbons is preferably used.

In some preferred embodiments, the PSA contains 1 to 60 parts by mass of the organic liquid component relative to 100 parts by mass of the water-dispersed copolymer. Setting of the amount of the organic liquid component in this range can preferably combine reduction of skin irritation in peeling and good water resistance.

The specification also provides a medical PSA sheet. The PSA sheet has any of medical PSAs disclosed herein on at least one side of a support. A PSA sheet having such a configuration may be preferably used in the forms of various medical PSA sheets such as adhesive bandages, surgical tapes, first-aid adhesive tapes, large adhesive tapes, dressing materials, and cataplasm materials.

### [Brief Description of Drawings]

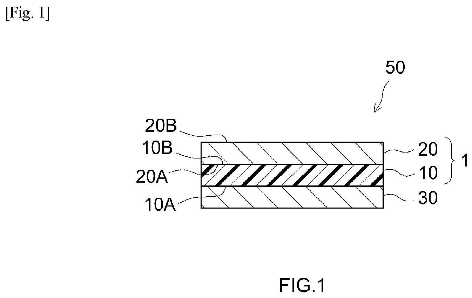

Fig. 1 is a cross-sectional view schematically illustrating a PSA sheet according to an embodiment.

### [Description of Embodiments]

Preferred embodiments of the present invention will now be described below. Matters that are other than those particularly mentioned herein but are necessary to practice the present invention can be understood by a person skilled in the art based on teaching for practice of the invention described herein and common technical knowledge at the time of filing. The present invention can be implemented based on the contents disclosed herein and common technical knowledge in the art. In the drawings below, members or sites producing the same effects may be described with a common reference numeral, and duplicated descriptions may be omitted or simplified. The embodiments depicted in the drawings are schematized for explicitly illustrating the present invention, and do not necessarily represent the accurate size or reduction scale of a product actually provided.

As used herein, the term "PSA" refers to, as described earlier, a material that is in a soft solid form (a viscoelastic material) at around room temperature and has a property to adhere easily to an adherend with some pressure applied. As defined in *"Adhesion: Fundamentals and Practice"* by C. A. Dahlquist, McLaren & Sons (1966), P. 143, the PSA referred to herein can be a material that has a property satisfying complex tensile modulus E* (1Hz) < 10⁷ dyne/cm² (typically, a material that has the described characteristics at 25 °C).

In addition, as used herein, the term "weight" may be construed as "mass". For example, the term "% by weight" may be construed as "% by mass, and the term "parts by weight" may be construed as "parts by mass".

### <PSA Composition>

### (Water-dispersed Copolymer)

The PSA composition disclosed herein contains a water-dispersed copolymer. The term "water-dispersed copolymer" here refers to a copolymer synthesized in a water-dispersed form, typically to a copolymer synthesized by emulsion polymerization. The use of the water-dispersed copolymer as a PSA forming component can lead to independence from use of an organic solvent or reduction in the amount of an organic solvent, and form a PSA with consideration for environment, health of an operator, and the like. The water-dispersed copolymer is typically contained as a base polymer in the PSA composition. The "base polymer" here refers to a primary component among polymers in the PSA composition (which can also be a PSA) and is not to be construed as being limited beyond this definition. A "primary component" as used herein refers to a component present in the largest proportion on a weight basis among components included. Accordingly, for example, when the PSA composition contains three or more kinds of polymers, the amount of the primary component among the polymers is 34% by weight or more, and may typically be more than 50% by weight.

As the water-dispersed copolymer, an acrylic copolymer is used. An acrylic PSA containing an acrylic copolymer is likely to provide good adhesiveness, also relatively less irritant to skin than a rubber-based PSA, and thus preferred as a medical PSA to be attached on skin. As used herein, the term "acrylic copolymer" refers to a polymer derived from a monomer mixture containing more than 50 % by weight of an acrylic monomer. The acrylic monomer refers to a monomer having at least one (meth)acryloyl group in a molecule. As used herein, the term "(meth)acryloyl" comprehensively refers to acryloyl and methacryloyl. Similarly, the term "(meth)acrylate" comprehensively refers to acrylate and methacrylate, and the term "(meth)acryl" comprehensively refers to acryl and methacryl.

In some embodiments, as the water-dispersed copolymer, a polymer of a monomer mixture containing an alkyl (meth)acrylate (m1) as a primary component is used. For example, the PSA composition preferably contains, as a base polymer, a copolymer formed from a monomer mixture containing an alkyl (meth)acrylate having a linear or branched alkyl group with one or more and 20 or less carbon atoms at the ester terminus. Hereinafter, an alkyl (meth)acrylate having, at the ester terminus, an alkyl group with X or more and Y or less carbon atoms may be represented as an "C_{X-Y} alkyl (meth)acrylate." In some preferred embodiments, in view of obtaining desired adhesive properties (e.g., adhesive strength and cohesive strength), the amount of the alkyl (meth)acrylate (m1) in the monomer mixture is suitably above 50% by weight, preferably 70 % by weight or more, more preferably 80% by weight or more, even more preferably 90% by weight or more, and may be, e.g., 92% by weight or more, or even 94% by weight or more. In view of obtaining the below-described effects based on copolymerization of a carboxy group-containing monomer (m2) and an N-containing monomer (m3), the amount of the alkyl (meth)acrylate (m1) in the monomer mixture is, e.g., 99.4% by weight or less, preferably 99% by weight or less, more preferably 98% by weight or less, even more preferably 97% by weight or less. The alkyl (meth)acrylate (m1) can be used singly or in combination of two or more kinds thereof.

Non-limiting specific examples of the C₁₋₂₀ alkyl (meth)acrylate include methyl (meth)acrylate, ethyl (meth)acrylate, propyl (meth)acrylate, isopropyl (meth)acrylate, n-butyl (meth)acrylate, isobutyl (meth)acrylate, s-butyl (meth)acrylate, t-butyl (meth)acrylate, pentyl (meth)acrylate, isopentyl (meth)acrylate, hexyl (meth)acrylate, heptyl (meth)acrylate, octyl (meth)acrylate, 2-ethylhexyl (meth)acrylate, isooctyl (meth)acrylate, nonyl (meth)acrylate, isononyl (meth)acrylate, decyl (meth)acrylate, isodecyl (meth)acrylate, undecyl (meth)acrylate, dodecyl (meth)acrylate, tridecyl (meth)acrylate, tetradecyl (meth)acrylate, pentadecyl (meth)acrylate, hexadecyl (meth)acrylate, heptadecyl (meth)acrylate, stearyl (meth)acrylate, isostearyl (meth)acrylate, nonadecyl (meth)acrylate, and eicosyl (meth)acrylate.

In some embodiments, among the C₁₋₂₀ alkyl (meth)acrylates, it is preferred to use a C₄₋₂₀ alkyl (meth)acrylate, more preferred to use a C₄₋₁₈ alkyl (meth)acrylate, even more preferred to use a C₄₋₁₂ alkyl (meth)acrylate, or particularly preferred to use a C₄₋₁₀ alkyl (meth)acrylate. In some embodiments, in the monomer mixture, the amount of the C₄₋₁₈ alkyl (meth)acrylate (preferably C₄₋₁₂ alkyl (meth)acrylate, more preferably C₄₋₁₀ alkyl (meth)acrylate) in the C₁₋₂₀ alkyl (meth)acrylate is preferably 50% by weight or more, more preferably 70% by weight or more, even more preferably 90% by weight or more, or may be 95% by weight or more, or 98 to 100% by weight or more. The use of such an alkyl (meth)acrylate as a primary component helps obtain good adhesive properties.

In some preferred embodiments, a C₇₋₉ alkyl acrylate is used as the C₁₋₂₀ alkyl (meth)acrylate. The use of a C₇₋₉ alkyl acrylate can preferably provide a PSA with good adhesive strength and excellent flexibility. A PSA with high flexibility is likely to provide high tight adhesiveness to skin, which has a complicated surface shape that is expandable and deformable. In some preferred embodiments, in the monomer mixture, the amount of a C₇₋₉ alkyl acrylate in the C₁₋₂₀ alkyl (meth)acrylate is, e.g., suitably 50% by weight or more, more preferably 70% by weight or more, even more preferably 90% by weight or more, and may be 95% by weight or more, or 98 to 100% by weight. For example, 2-ethylhexyl acrylate (2EHA) is particularly preferably contained in the monomer mixture. Other examples of a C₇₋₉ alkyl acrylate potentially preferably used include heptyl acrylate, octyl acrylate, isooctyl acrylate and isononyl acrylate.

The monomer mixture contains a carboxy group-containing monomer (m2) and an N-containing monomer (m3). Combined use of the monomers (m2) and (m3) can improve water resistance. In addition, since the combined use can provide high adhesive strength, the composition containing the organic liquid component described later can maintain good tight adhesiveness to skin. This may be because coexistence of the carboxy group-containing monomer (m2) and the N-containing monomer (m3) leads to improved cohesive strength based on acid-base interaction therebetween, and the cohesive strength contributes to increase in water resistance and adhesive strength. It should be noted that the art disclosed herein is not limited to this consideration. By utilizing the action based on the combined use of the carboxy group-containing monomer (m2) and the N-containing monomer (m3), combination of flexibility, water resistance, and adhesive strength can be achieved with the use of e.g., a highly flexible material as the alkyl (meth)acrylate (m1).

The carboxy group-containing monomer (m2) is not particularly limited, and various monomer having a carboxy group can be used. The carboxy group-containing monomer (m2) can be used singly or in combination of two or more kinds thereof.

In some embodiments, the carboxy group-containing monomer (m2), which is used in the monomer mixture, contains an acrylic acid (m2a). By using the acrylic acid (m2a) as the carboxy group-containing monomer (m2) in synthesis of the water-dispersed copolymer, the acrylic acid (m2a), which has relatively higher polarity, in a polymeric particle in a dispersion is present close to the surface of the polymeric particle and contributes to dispersive stability of particles and ease of preparing a dispersion as well as interacts with the N-containing monomer (m3). It should be noted that the art disclosed herein is not limited to this consideration.

In an embodiment of using the acrylic acid (m2a) as the carboxy group-containing monomer (m2), the amount of the acrylic acid (m2a) (in other words, a copolymerization proportion of the acrylic acid (m2a) in the water-dispersed copolymer) is not particularly limited, may be, e.g., 0.1% by weight or more, and is preferably 0.5% by weight or more, more preferably 1.0% by weight or more (e.g., 1.5% by weight or more) in a monomer mixture. Setting the amount of the acrylic acid (m2a) to a predetermined amount or more may lead to improved adhesive properties (e.g., adhesive strength and cohesive strength), dispersive stability, ease of preparing a dispersion. In some embodiments, the amount of the acrylic acid (m2a) is, e.g., suitably 10% by weight or less, preferably 8.0% by weight or less, more preferably 6.0% by weight or less, even more preferably 5.0% by weight or less, particularly preferably 3.0% by weight or less in the monomer mixture, in view of water resistance, flexibility, and other properties.

In some preferred embodiments, the carboxy group-containing monomer (m2) contains the acrylic acid (m2a) and a carboxy group-containing monomer (m2b) that is more hydrophobic than the acrylic acid (m2a). Combined use of the acrylic acid (m2a) and the carboxy group-containing monomer (m2b), which has relatively high hydrophobicity, as the carboxy group-containing monomer (m2) can preferably provide a PSA having excellent water resistance and adhesive strength. In synthesis of the water-dispersed copolymer, while the acrylic acid (m2a), which has relatively higher polarity, in a polymeric particle in a dispersion is present close to the surface of the polymeric particle as described above, the carboxy group-containing monomer (m2b), which has relatively higher hydrophobicity, may be majorly present inside the polymeric particle by virtue of the hydrophobicity and improve cohesive strength inside the polymeric particle on the basis of acid-base interaction with the N-containing monomer (m3). Consequently, after formation of a PSA via fusion of polymeric particles by subsequent drying, the PSA may still have high cohesive strength in a bulk form and exert excellent water resistance and adhesive strength. It should be noted that the art disclosed herein is not limited to this consideration. Whether the carboxy group-containing monomer (m2b) is more hydrophobic than the acrylic acid (m2a) can be determined by chemical structures, and can also be determined by the water solubility at 20°C described later.

Examples of the carboxy group-containing monomer (m2b), which is more hydrophobic than the acrylic acid (m2a), include ethylenic unsaturated monocarboxylic acids such as methacrylic acid (MAA), carboxyethyl (meth)acrylate, carboxypentyl (meth)acrylate, 2-(meth)acryloyloxyethyl hexahydrophthalic acid, 2-(meth)acryloyloxypropyl hexahydrophthalic acid, 2-(meth)acryloyloxyethyl phthalic acid, 2-(meth)acryloyloxyethyl succinic acid, 2-(meth)acryloyloxyethyl-2-hydroxyethylphthalic acid, 2-(meth)acryloyloxyethyl maleic acid, carboxypolycaprolactone mono(meth)acrylate, 2-(meth)acryloyloxyethyl tetrahydrophthalic acid, crotonic acid and isocrotonic acid. The carboxy group-containing monomer (m2b) can be used singly or in combination of two or more kinds thereof. In particular, MAA is preferred in view of being effective in a small amounts owing to its low molecular weight.

In some preferred embodiments, a monomer having a water solubility (solubility to water) of 15.0 g/100 mL or less at 20°C is used as the carboxy group-containing monomer (m2b), which is more hydrophobic than the acrylic acid (m2a). Combined use of the acrylic acid (m2a) and the carboxy group-containing monomer (m2b), which has hydrophobicity with a water solubility equal to or less than a predetermined value as described above may cause the monomer (m2b) to be suitably present inside a polymeric particle and contribute to improved cohesive strength inside the particle. In view of improving cohesive strength inside a polymeric particle, the water solubility of the carboxy group-containing monomer (m2b) is preferably 12.0 g/100 mL or less, and may be 10.0 g/100 mL or less, 8.0 g/100 mL or less, or 5.0 g/100 mL or less. In view of ease of emulsion polymerization, the water solubility of the carboxy group-containing monomer (m2b) is preferably 2.0 g/100 mL or more, and may be 6.0 g/100 mL or more, or 8.0 g/100 mL or more. Preferred examples of the carboxy group-containing monomer (m2b) with such water solubility include MAA (water solubility at 20°C: 9.7 g/100 mL). As a water solubility of a monomer material at 20°C, a value measured on the basis of Techniques of Chemistry 4th Ed. Vol. II Organic Solvents (1985) : New York, NY. John Wiley and Sons, Inc. ICSC(J) 1997 can be used.

In an embodiment in which the acrylic acid (m2a) and the carboxy group-containing monomer (m2b), which is more hydrophobic than the acrylic acid (m2a), are used in combination as the carboxy group-containing monomer (m2), a usage ratio of both is not particularly limited, and a suitable ratio can be employed corresponding to dispersive stability, ease of preparing a polymer, the kind of the carboxy group-containing monomer (m2b) used, and the like. In some embodiments, in view of dispersive stability, ease of preparing a dispersion, or other properties, a ratio (m2a/m2b) of the amount of the acrylic acid (m2a) to the amount of the carboxy group-containing monomer (m2b), which is more hydrophobic than the acrylic acid (m2a) on a weight basis, may be, e.g., 0.1 or more, 0.5 or more, 1 or more (e.g., more than 1), 1.5 or more, or 1.8 or more. In some embodiments, in view of improving water resistance and adhesive strength, the ratio (m2a/m2b) may be, e.g., 10 or less, 5 or less, 3 or less, or 2 or less.

In an embodiment in which the carboxy group-containing monomer (m2b), which is more hydrophobic than the acrylic acid (m2a), is used as the carboxy group-containing monomer (m2), the amount of the carboxy group-containing monomer (m2b), which is more hydrophobic than the acrylic acid (m2a) (in other words, a copolymerization proportion of the carboxy group-containing monomer (m2b) in the water-dispersed copolymer) is not particularly limited, and may be, e.g., 0.1% by weight or more, and is preferably 0.3% by weight or more, more preferably 0.7% by weight or more, and may be 1.0% by weight or more in the monomer mixture. Increase in the amount of the carboxy group-containing monomer (m2b) in a predetermined range can preferably improve cohesive strength, water resistance, and adhesive strength. In some embodiments, in view of water resistance, flexibility, and other properties, the amount of the carboxy group-containing monomer (m2b) is, e.g., suitably 10% by weight or less, preferably 8.0% by weight or less, more preferably 6.0% by weight or less, even more preferably 5.0% by weight or less, particularly preferably 3.0% by weight or less, and may be, e.g. 1.5% by weight or less in the monomer mixture.

The amount of the carboxy group-containing monomer (m2) (in other words, a copolymerization proportion of the carboxy group-containing monomer (m2) in the water-dispersed copolymer) is not particularly limited, and may be, e.g., 0.1% by weight or more, and is preferably 0.5% by weight or more, more preferably 1.0% by weight or more, even more preferably 2.0% by weight or more, particularly preferably 2.5% by weight or more, and may be 3.0% by weight or more in the monomer mixture. Increase in the amount of the carboxy group-containing monomer (m2) in a predetermined range can preferably improve adhesive strength, cohesive strength, and water resistance. In some embodiments, in view of water resistance, flexibility, and other properties, the amount of the carboxy group-containing monomer (m2) is, e.g., suitably 10% by weight or less, preferably 8.0% by weight or less, more preferably 6.0% by weight or less, even more preferably 5.0% by weight or less, and may be 4.5% by weight or less, or 3.5% by weight or less in the monomer mixture.

The N-containing monomer (m3) is not particularly limited, and various monomer having an nitrogen atom can be used. For example, a monomer having an N-containing ring, an amide group-containing monomer, and an amino group-containing monomer can be used. The N-containing monomer (m3) can be used singly or in combination of two or more kinds thereof.

Examples of the monomer having an N-containing ring include *N*-vinyl-2-pyrrolidone (NVP), *N*-methylvinylpyrrolidone, *N*-vinylpyridine, *N*-vinylpiperidone, *N*-vinylpyrimidine, *N-*vinylpiperazine, *N*-vinylpyrazine, *N*-vinylpyrrole, *N*-vinylimidazole, *N*-vinyloxazole, *N-*(meth)acryloyl-2-pyrrolidone, *N*-(meth)acryloyl piperidine, *N*-(meth)acryloylpyrrolidine, *N-*(meth)acryloylmorpholine, *N*-vinylmorpholine, *N*-vinyl-3-morpholinone, N*-*vinyl-2-caprolactam, *N-*vinyl-1,3-oxadin-2-one, *N*-vinyl-3,5-morpholindione, *N*-vinylpyrazole, *N*-vinylisoxazole, *N-*vinylthiazole, *N*-vinylisothiazole and *N*-vinylpyridazine.

Examples of the amide group-containing monomer include (meth)acrylamide; *N,N*-dialkyl (meth)acrylamides such as *N,N*-dimethyl (meth)acrylamide, *N,N*-diethyl (meth)acrylamide, *N,N-*dipropyl (meth)acrylamide, *N,N*-diisopropyl (meth)acrylamide, *N,N*-di(*n-*butyl) (meth)acrylamide, and *N,N*-di(t-butyl) (meth)acrylamide; N-alkyl (meth)acrylamides such as *N*-ethyl (meth)acrylamide, *N*-isopropyl (meth)acrylamide, *N-*butyl (meth)acrylamide, and *N*-n-butyl (meth)acrylamide; *N*-vinyl carboxamides such as *N*-vinylacetamide; monomers having a hydroxy group and an amide group, e.g., *N*-hydroxyalkyl (meth)acrylamides such as *N*-(2-hydroxyethyl) (meth)acrylamide, *N*-(2-hydroxypropyl) (meth)acrylamide, *N*-(1-hydroxypropyl) (meth)acrylamide, *N*-(3-hydroxypropyl) (meth)acrylamide, *N*-(2-hydroxybutyl) (meth)acrylamide, *N*-(3-hydroxybutyl) (meth)acrylamide, and *N*-(4-hydroxybutyl) (meth)acrylamide; monomers having an alkoxy group and an amide group, e.g., *N*-alkoxyalkyl (meth)acrylamide such as *N*-methoxymethyl (meth)acrylamide, *N*-methoxyethyl (meth)acrylamide, and *N*-butoxymethyl (meth)acrylamide; and other monomers such as *N,N*-dimethylaminopropyl (meth)acrylamide.

Examples of the amino group-containing monomer include *N,N-*dialkyl aminoalkyl (meth)acrylate such as *N,N*-dimethylaminoethyl (meth)acrylate; *N*-alkyl (meth)acrylate such as aminoethyl (meth)acrylate; and other monomers such as *t*-butyl aminoethyl (meth)acrylate.

In some preferred embodiments, a monomer having a structure in which a nitrogen atom is bound to no hydrogen atoms is used as the N-containing monomer (m3). Combined use of a N-containing monomer having the structure and the carboxy group-containing monomer (m2) can improve water resistance. The combined use can provide high adhesive strength, and thus the composition containing the organic liquid component described later can maintain good tight adhesiveness to skin. A N-containing monomer having a nitrogen atom bound to no hydrogen atoms may lead to stronger acid-base interaction with carboxylic acid, and cohesive strength is improved on the basis of such an acid-base interaction, thereby increasing water resistance and adhesive strength. It should be noted that the art disclosed herein is not limited to this consideration. A N-containing monomer with a structure having a nitrogen atom bound to no hydrogen atom can be used singly or in combination of two or more kinds thereof.

With no particular limitation, a N-containing monomer having a low acid dissociation constant (pKa) is preferably used as the N-containing monomer (m3). A lower pKa may lead to a stronger acid-base interaction with carboxylic acid and be more likely to provide excellent cohesive strength. The pKa of the N-containing monomer (m3) may be, e.g., 7 or less, 5 or less, or 3 or less. In some preferred embodiments, in view of providing more excellent water resistance and adhesive strength, the N-containing monomer (m3) with a pKa of 1 or less is used. The pKa is more preferably 0.5 or less, even more preferably 0 or less (e.g., less than 0), and may be -0.5 or less. The lower limit of the pKa of the N-containing monomer (m3) is -2 or more, and may be -1.5 or more, or -1 or more. The use of the N-containing monomer (m3) with a pKa in this range may provide good acid-base interaction on the basis of its basicity. Preferred examples of a N-containing monomer having such a pKa include NVP (pKa: -0.34). It should be noted that a pKa for a substance to be measured can be calculated by measuring a hydrogen-ion concentration with a pH meter and using the concentration of the substance and the hydrogen-ion concentration.

In some preferred embodiments, a monomer having a cyclic structure containing a nitrogen atom in a ring (a monomer having an N-containing ring) is used as the N-containing monomer (m3). The use of a monomer having an N-containing ring can achieve more excellent water resistance and adhesive strength. This may be because a structure having an N-containing ring produces a distance to easily generate interaction between acid and base, thereby being likely to provide an effect of improving more excellent cohesive strength. Preferred examples of a monomer having an N-containing ring include NVP and N-(meth)acryloylmorpholine. In particular, NVP has good biocompatibility, and is favorably applicable to a medical PSA to be attached to skin. A monomer having an N-containing ring can be used singly or in combination of two or more kinds thereof.

In an embodiment in which a monomer having an N-containing ring is used as the N-containing monomer (m3), the amount of the monomer having an N-containing ring in a whole of the N-containing monomer (m3) is not particularly limited. In some embodiments, in view of effectively exerting an effect of use of a monomer having an N-containing ring, the amount of an monomer having an N-containing ring in a whole of the N-containing monomer (m3) may be, e.g., 10% by weight or more, and is suitably 50% by weight or more, preferably 70% by weight or more, and may be 90% by weight or more (e.g., 95 to 100% by weight).

The amount of the N-containing monomer (m3) (in other words, a copolymerization proportion of the N-containing monomer (m3) in the water-dispersed copolymer) is not particularly limited, and may be, e.g., 0.1% by weight or more, preferably 0.3% by weight or more, more preferably 0.5% by weight or more, even more preferably 0.8% by weight or more, particularly preferably 1.0% by weight or more in the monomer mixture. Increase in the amount of the N-containing monomer (m3) in a predetermined range can preferably improve cohesive strength, water resistance, and adhesive strength. In some embodiments, in view of water resistance, flexibility, and other properties, the amount of the N-containing monomer (m3) is, e.g., suitably 10% by weight or less, preferably 8.0% by weight or less, more preferably 6.0% by weight or less, yet more preferably 5.0% by weight or less, even more preferably 3.0% by weight or less, particularly preferably 2.5% by weight or less, and may be 2.0% by weight or less, 1.5% by weight or less, or 1.2% by weight or less in the monomer mixture.

A usage ratio of the carboxy group-containing monomer (m2) to the N-containing monomer (m3) is not particularly limited, and appropriately set so as to exert desired water resistance, adhesive strength, or other properties. In some embodiments, a ratio (m2/m3) of the amount of the carboxy group-containing monomer (m2) to the amount of the N-containing monomer (m3) on a weight basis may be, e.g., 0.1 or more, and is suitably 0.5 or more, preferably 1 or more (e.g., more than 1), more preferably 1.5 or more, even more preferably 2.0 or more, and may be 2.5 or more. In some embodiments, the ratio (m2/m3) may be, e.g., 10 or less, and is suitably 7 or less, preferably 5 or less, more preferably 4 or less, and may be 3 or less.

The monomer mixture further contains a silane-based monomer (m4). With having a monomer composition containing the silane-based monomer (m4), the water-dispersed copolymer allows introduction of a cross-linked structure derived by a condensation reaction of silanol groups (silanol condensation) between polymeric particles in a PSA formed, and such a cross link between polymeric particles can increase cohesive strength, and improve water resistance and adhesive strength. The silane-based monomer (m4) is also referred to as a silane coupling agent.

As the silane-based monomer (m4), an alkoxysilyl group-containing monomer is preferably used. An alkoxysilyl group-containing monomer is typically a ethylenic unsaturated monomer having at least one (preferably two or more, e.g., two or three) alkoxysilyl group in a molecule. Examples of the silane-based monomer (m4) include 3-(meth)acryloxypropyltrimethoxysilane, 3-(meth)acryloxypropyltriethoxysilane, 3-(meth)acryloxypropylmethyldimethoxysilane, and 3-(meth)acryloxypropylmethyldiethoxysilane. The silane-based monomer (m4) can be used singly or in combination of two or more kinds thereof.

The amount of the silane-based monomer (m4) in the monomer mixture is, e.g., suitably 0.001% by weight or more, preferably 0.01% by weight or more. In some embodiments, in view of improving tight adhesiveness to an adherend, the amount of the silane-based monomer (m4) is, e.g., suitably 0.5% by weight or less, preferably 0.2% by weight or less, and may be 0.1% by weight or less, or 0.05% by weight or less.

The monomer mixture may optionally contain another monomer that is different from the carboxy group-containing monomer (m2), the N-containing monomer (m3), and the silane-based monomer (m4) and copolymerizable with any of these monomers (another copolymerizable monomer). As the another copolymerizable monomer, a monomer having a functional group such as a hydroxy group, a monomer whose homopolymer having relatively high glass transition temperature (e.g., 10°C or higher) and the like can be preferably used. The another copolymerizable monomer can be used singly or in combination of two or more kinds thereof.

Non-limiting examples of the another copolymerizable monomer include acid anhydride group-containing monomers, hydroxy group-containing monomers (e.g., 2-hydroxyethyl acrylate and 4-hydroxybutyl acrylate), sulfonate or phosphate group-containing monomers, epoxy group-containing monomers, cyano group-containing monomers, isocyanate group-containing monomers, alkoxy group-containing monomers, vinyl esters, vinyl ethers, aromatic vinyl compounds, olefins, (meth)acrylates having alicyclic hydrocarbon groups, (meth)acrylates having aromatic hydrocarbon groups; as well as heteroring-containing (meth)acrylates such as tetrahydrofurfuryl (meth)acrylate, halogen atom-containing (meth)acrylates such as vinyl chloride and fluorine atom-containing (meth)acrylates, silicon atom-containing (meth)acrylates such as silicone (meth)acrylate, (meth)acrylic esters derived from terpene compound derivative alcohols, and polyfunctional monomers (monomers having at least two polymerizable functional groups with unsaturated double bonds such as (meth)acryloyl groups and vinyl groups).

The another copolymerizable monomer can be used in an appropriate amount as far as it does not hinder an effect of the present invention. When the monomer mixture contains the another copolymerizable monomer, the amount of the another copolymerizable monomer in the monomer mixture is suitably approximately 30% by weight or less, and may be 10% by weight or less, 3% by weight or less, or 1% by weight or less (e.g., 0.1% by weight or less), and the monomer mixture may be substantially no another copolymerizable monomer. In addition, the PSA disclosed herein can achieve good water resistance on the basis of high cohesive strength by combined use of the carboxy group-containing monomer (m2) and the N-containing monomer (m3), eliminating need for relying on a hydroxy group-containing monomer used for the purpose of inducing a cross-linking reaction, improving cohesive strength, and the like. Accordingly, the art disclosed herein can be preferably performed in an embodiment in which the content of a hydroxy group-containing monomer in the monomer mixture is 3% by weight or less (e.g., 1% by weight or less, furthermore 0.1% by weight or less), or in an embodiment in which the monomer mixture contains substantially no hydroxy group-containing monomer.

The water-dispersed copolymer can be synthesized by emulsion polymerization. The emulsion polymerization can prepare a polymerization liquid in an emulsion form where polymers are dispersed in water (a polymer emulsion; also referred to as a water dispersion of polymer). An embodiment of the emulsion polymerization is not particularly limited and can be performed by appropriately employing various monomer supplying methods, polymerization conditions, materials used, and the like similar to those of heretofore known typical emulsion polymerization. Examples of the monomer supplying methods that may be appropriately employed include an all-at-once approach where a whole monomer mixture is supplied in one portion, a continuous (dropwise) supplying approach, and a portion-wise (dropwise) supplying approach. The monomer mixture is preferably supplied as a dispersion (emulsion) of a portion or all thereof emulsified in water, and for example, supply of the monomer mixture by dropwise approach is preferably performed by dropwise in an emulsified form. Polymerization temperature can be, e.g., about 30°C or higher (regularly 50°C or higher), or suitably about 100°C or lower (regularly 80 °C or lower).

An initiator used in polymerization can be appropriately selected among heretofore known polymerization initiators corresponding to a polymerization method. For examples, an azo-based polymerization initiator, a peroxide-based polymerization initiator, a redox-based polymerization initiator with combination of a peroxide and a reducing agent, and a substituted ethane-based polymerization initiator can be used. In the emulsion polymerization, a water-soluble polymerization initiator is preferably used. A polymerization initiator can be used singly or in appropriate combination of two or more kinds thereof. The amount of a polymerization initiator can be selected, e.g., in the range of approximately 0.005 to 1 part by weight (typically approximately 0.01 to 1 part by weight) relative to 100 parts by weight of the monomer mixture).

An emulsifier used in the emulsion polymerization is not particularly limited, and known anionic surfactants, nonionic surfactants and the like can be used. In some embodiments, in view of improving water resistance, a surfactant (reactive emulsifier) having a radically-polymerizable functional group is preferably used. A emulsifier can be used singly or in combination of two or more kinds thereof.

Examples of the reactive emulsifier usable include an anionic or nonionic surfactant and the like having a structure in which a radically-polymerizable functional group is introduced. In view of polymerization stability, emulsifiability, and the like during emulsion polymerization, an anionic reactive emulsifier is preferably used. Examples of the radically-polymerizable functional group include a vinyl group, propenyl group, isopropenyl group, vinyl ether group (vinyloxy group), and allyl ether group (allyloxy group). The concept of a propenyl group here encompasses a 1-propenyl group (CH₃-CH=CH-) and 2-propenyl group (CH₂=CH-CH₂-, which may also be referred to as an allyl group).

Examples of the anionic reactive emulsifier include polyoxyethylene (allyloxymethyl) alkyl ether sulfates (e.g., ammonium salts), polyoxyethylene nonyl propenyl phenyl ether sulfates (e.g., ammonium salts), alkyl allyl sulfosuccinates (e.g., sodium salts), methacryloxy polyoxypropylene sulfuric acid ester salts (e.g., sodium salts), and polyoxyalkylene alkenyl ether sulfates (e.g., ammonium salts in which the alkenyl group terminal is an isopropenyl group). When the anionic reactive emulsifier is in a salt form, the salt may be, e.g., a metal salt such as a sodium salt, or a non-metal salt such as an ammonium salt or an amine salt.

Examples of the nonionic reactive emulsifier include polyoxyethylene nonyl propenyl phenyl ether.

The monomer mixture may be subjected to emulsion polymerization in the presence of a reactive emulsifier having a radically-polymerizable functional group, thereby reacting the reactive emulsifier to be incorporated in an polymer. The reactive emulsifier incorporated in a polymer is restricted in its movement within a PSA layer, and thus is less likely to migrate to a surface of the PSA layer. Accordingly, the use of a reactive emulsifier can inhibit migration of the emulsifier to a surface of the PSA layer. This may be advantageous in suppressing decrease in water-resistant adhesive strength when water is generated on a surface of the PSA layer surface.

In emulsion polymerization, the amount of an emulsifier relative to 100 parts by weight of the monomer mixture may be, e.g., 0.2 parts by weight or more, 0.5 parts by weight or more, or 1.0 part by weight or more, or 1.5 parts by weight or more. In view of improving water resistance, in some embodiments, the amount of an emulsifier relative to 100 parts by weight of the monomer mixture is suitably 10 parts by weight or less, preferably 5 parts by weight or less, more preferably 3 parts by weight or less, even more preferably 2.5 parts by weight or less

In the polymerization, various kinds of heretofore known chain transfer agents (which may also be recognized as molecular weight-adjusting agents or polymerization degree-adjusting agents) can be used optionally. As a chain transfer agent, mercaptan can be used, such as *n-*dodecyl mercaptan, t-dodecyl mercaptan (t-lauryl mercaptan), thioglycolic acid and α-thioglycerol. Alternatively, a chain transfer agent with no sulfur atom (a sulfur-free chain transfer agent) can be used. A chain transfer agent can be used singly or in combination of two or more kinds thereof. In use of a chain transfer agent, the amount can be, e.g., approximately 0.01 to 1 part by weight relative to 100 parts by weight of the monomer mixture.

### (Organic Liquid Component)

The PSA composition disclosed herein contains an organic liquid component. The organic liquid component is defined as an organic component that exhibits a liquid form at normal temperature (23°C). As the organic liquid component, a compound having more than one hydroxy group is used. A PSA containing the organic liquid component having such a chemical structure is less irritant to skin in peeling from the skin, has good water resistance, and further allows prevention of pollution of skin by a PSA component. In particular, the organic liquid component, which contains more than one hydroxy group and has a suitable hydrophilicity, may be well arranged on a surface of a polymeric particle in a PSA, and plasticizes the PSA wholly and suitably as well as forms a weak boundary layer (WBL) on a surface of a PSA layer, thereby reducing irritation to skin in peeling. The action of the organic liquid component may be exerted along with retention of cohesive strength of a PSA, and the PSA has good water resistance and provides prevention of pollution of skin by a PSA component due to cohesive failure in peeling, on the basis of cohesive strength by the water-dispersed copolymer. Furthermore, addition of the organic liquid component leads to a certain degree of reduction in adhesive strength of a PSA, but the PSA has improved tight adhesiveness to skin by virtue of suitable plasticization as described above, thus allowing combination of good tight adhesiveness to skin and reduction of skin irritation in peeling. It should be noted that the art disclosed herein is not limited to this consideration. The organic liquid component can be used singly or in combination of two or more kinds thereof.

The number of hydroxy groups in a molecule of the organic liquid component is more than one, preferably 2 or more, more preferably 3 or more. As the organic liquid component has a greater number of hydroxy groups, compatibility with a surface of a polymeric particle in a PSA tend to be improved. Furthermore, the number of hydroxy groups in a molecule of the organic liquid component is suitably within an appropriate range on the basis of a balance with a hydrophobic structure in the organic liquid component, and may be, e.g., 20 or less, 15 or less, 10 or less , and in some preferred embodiments, it may be 5 or less, or 4 or less. When the organic liquid component is formed of two or more kinds having different chemical structures, the number of hydroxy groups in a molecule can be derived as a weighted average efficiency from a sum of products of the number of hydroxy groups of each component corresponding to the organic liquid component, and a weight fraction of a corresponding component.

In some embodiments, a surfactant is preferably used as the organic liquid component. For example, a non-ionic surfactant, an anionic surfactants, a cationic surfactants or the like can be used as the organic liquid component. In particular, a non-ionic surfactant is preferred. The use of a suitable surfactant as the organic liquid component can preferably combine reduction of skin irritation in peeling and good water resistance.

A HLB of the organic liquid component (typically, a surfactant) is not particularly limited. In some embodiments, in view of preventing skin irritation, the HLB is preferably 3 or more, more preferably 5 or more, yet more preferably 6 or more, even more preferably 7 or more, particularly preferably 8 or more. With a HLB in this range, the organic liquid component can achieve an excellent effect of reducing skin irritation by being used in combination with the water-dispersed copolymer disclosed herein. With this HLB, the organic liquid component is likely to provide excellent prevention of pollution of skin. The upper limit of the HLB is 20 or less, and in some preferred embodiments, in view of water resistance, it is 18 or less, more preferably 16 or less, and may be 14 or less, 12 or less, 10 or less, or 9 or less. The use of a substance having an appropriate HLB in the range as the organic liquid component can better combine reduction of skin irritation in peeling and good water resistance. With the HLB, the organic liquid component is likely to provide excellent prevention of pollution of skin.

A HLB herein refers to the hydrophile-lipophile balance by Griffin, which is a value indicating the degree of compatibility of a surfactant with water, oil and the like and is represented by a numerical value of a ratio of hydrophilicity to lipophilicity between 0 to 20. A definition of HLB is as described in, e.g., W. C. Griffin: J. Soc. Cosmetic Chemists, 1,311(1949) or Takahashi, Koshitami, Yoshiro Namba, Motoo Koike, and Masao Kobayashi eds., "Surfactant Handbook", 3rd edition, published by Kogyo Tosho Co., Ltd, November 25, 1972, p179-182.

In some embodiments, a fatty acid ester derivative of polyhydric alcohol (that is a concept encompassing fatty acid esters of polyhydric alcohol and derivatives thereof) is preferably used as the organic liquid component. When having this structure, the organic liquid component has more than one hydroxy group and tends to exhibit a liquid form at normal temperature, and thus is likely to preferably exert desired action inside or on a surface of a PSA. As the polyhydric alcohol, tri- or more multivalent polyhydric alcohol such as sugar alcohol can be used. Non limiting examples of the polyhydric alcohol include glycerin, polyglycerol, trimethylolpropane, pentaerythritol, sorbitol, and sucrose. In particular, polyhydric alcohol to form a fatty acid ester derivative of the polyhydric alcohol is preferably glycerin, polyglycerol, and sorbitol, particularly preferably sorbitol. Sorbitol has a considerable number of hydroxy groups proximal to one another in a molecule. By hydrogen bonds between these hydroxy groups and carboxy groups on a surface of a polymeric particle, the organic liquid component with a structure derived from sorbitol may be well arranged on a surface of a polymeric particle in a PSA. It should be noted that the art disclosed herein is not limited to this consideration.

Fatty acid to form a fatty acid ester derivative of polyhydric alcohol is preferably a substance to provide the organic liquid component with suitable hydrophobicity, and is preferably, e.g., a fatty acid ester derivative of saturated fatty acid or unsaturated fatty acid having a carbon number in the range of 8 to 18. Unsaturated fatty acid having a relatively low melting point is likely to provide a substance exhibiting a liquid form even in the use of fatty acid having a large carbon number. The carbon number of the fatty acid may be 9 or more, 10 or more, 11 or more, or 12 or more. In view of having suitable hydrophilicity, the carbon number of the fatty acid is preferably 16 or less, more preferably 14 or less, even more preferably 12 or less. When formed from fatty acid having a carbon number in the range, the organic liquid component is likely to produce a PSA that imparts less damage such as exfoliation of keratin layers in peeling from skin, and more reduction of irritation to the skin. Non limiting examples of the fatty acid include lauric acid, myristic acid, palmitic acid, and oleic acid. In particular, lauric acid is preferred.

In some embodiments, the organic liquid component may have an oxyethylene unit (-CH₂-CH₂-O-), or may not have the oxyethylene unit. When having an oxyethylene unit, the organic liquid component can provide suitable hydrophilicity on a surface of a PSA, and further reduce more irritation to skin. When the organic liquid component with an oxyethylene unit is used, the number of oxyethylene units in the organic liquid component is 1 or more and may be 3 or more, and in view of improving hydrophilicity, it is preferably 5 or more, more preferably 10 or more, even more preferably 15 or more, and may be 18 or more. The upper limit of the number of oxyethylene units in the organic liquid component may be, e.g., 50 or less, and in view of water resistance, it is preferably 40 or less, more preferably 30 or less, even more preferably 25 or less. With no particular limitation, the oxyethylene unit may have a form of a polyoxyethylene (POE) chain containing the oxyethylene unit (a chain structure having 2 or more consecutive oxyethylene units) and be contained in the organic liquid component. For example, when the organic liquid component is a fatty acid ester derivative of polyhydric alcohol, the oxyethylene unit or POE chain may be contained in the form of it bound with polyhydric alcohol (ether bond) in the organic liquid component.

Non limiting examples of the organic liquid component include glycerol monooleate, polyglycerol laurate, polyglycerol oleate, polyglycerol myristate, sorbitan monolaurate, sorbitan monooleate, POE sorbitan monolaurate, POE sorbitan monopalmitate, POE sorbitan monostealate, and POE sorbitan monooleate.

The amount of the organic liquid component is not particularly limited. In some embodiments, the amount of the organic liquid component relative to 100 parts by weight of the water-dispersed copolymer may be 1 part by weight or more, or 10 parts by weight or more. The use of a predetermined amount or more of the organic liquid component can preferably exert an effect by inclusion of the organic liquid component. In some preferred embodiments, the amount of the organic liquid component relative to 100 parts by weight of the water-dispersed copolymer is 15 parts by weight or more, more preferably 20 parts by weight or more, even more preferably 25 parts by weight or more, and may be 30 parts by weight or more. In some embodiments, the amount of the organic liquid component relative to 100 parts by weight of the water-dispersed copolymer may be 100 parts by weight or less, or 80 parts by weight or less. Limitation of the amount of the organic liquid component facilitates good miscibility of the organic liquid component in a PSA and improvement in water resistance. In some preferred embodiments, the amount of the organic liquid component relative to 100 parts by weight of the water-dispersed copolymer is 60 parts by weight or less, more preferably 50 parts by weight or less, even more preferably 40 parts by weight or less, particularly preferably 35 parts by weight or less.

### (Crosslinking Agent)

The PSA composition disclosed herein may optionally contain a crosslinking agent. The type of the crosslinking agent is not particularly limited, and can be appropriately selected and used from heretofore known crosslinking agents. Examples of such a crosslinking agent include an isocyanate-based crosslinking agent, an epoxy-based crosslinking agent, an oxazoline-based crosslinking agent, an aziridine-based crosslinking agent, a melamine-based crosslinking agent, a peroxide-based crosslinking agent and a metal chelate-based crosslinking agent. A crosslinking agent can be used singly or in combination of two or more kinds thereof.

The amount of a crosslinking agent is not particularly limited, and can be selected, e.g., in the range of approximately 10 parts by weight or less (e.g., approximately 0.001 to 10 parts by weight, preferably approximately 0.01 to 5 parts by weight) relative to 100 parts by weight of the water-dispersed copolymer. In some embodiments, the amount of a crosslinking agent relative to 100 parts by weight of the water-dispersed copolymer is 1 part by weight or less, or may be 0.5 parts by weight or less, or 0.1 parts by weight or less (e.g., 0.05 parts by weight). In some embodiments, the PSA composition may be substantially free of a crosslinking agent. The art disclosed herein can provide high cohesive strength using a small amount of a crosslinking agent or substantially no crosslinking agent, by designing a monomer composition of the water-dispersed copolymer.

### (Other Optional Components)

The PSA composition disclosed herein may optionally contain a tackifier resin for the purpose of improving adhesive strength or the like. As the tackifier resin, one kind or two or more kinds selected from a phenol-based tackifier resin, a terpene-based tackifier resin, a modified terpene-based tackifier resin, a rosin-based tackifier resin, a hydrocarbon-based tackifier resin, an epoxy-based tackifier resin, a polyamide-based tackifier resin, an elastomer-based tackifier resin, and a ketone-based tackifier resin can be used. When the PSA composition contains a tackifier resin, the amount of the tackifier resin relative to 100 parts by weight of the water-dispersed copolymer is, e.g., suitably in the range of about 1 to 30 parts by weight, and may be 10 parts by weight or less. In some embodiments, the PSA composition contains substantially no tackifier resin. The art disclosed herein can provide the PSA composition (in turn, PSA) with good tight adhesiveness to skin, without containing a tackifier resin.

The PSA composition may optionally contain various additives generally used in the field of PSA compositions, such as a leveling agent, a crosslinking aid, a plasticizer, a softening agent, a filler, a colorant (a dye, a pigment), an anti-static agent, an anti-aging agent, a UV absorber, an antioxidant, a photo-stabilizer, or a dispersant. In regard to these various additives, heretofore known additives can be used by common methods; this does not particularly characterize the present invention, and detailed description will be omitted.

In some embodiments, the PSA composition may have a limited amount of component other than the water-dispersed copolymer and the organic liquid component. When containing a less amount of a component other than the water-dispersed copolymer and the organic liquid component, a PSA can provide prevention or reduction of an effect on skin caused by the component. For example, the PSA composition preferably has limitation in the use of a skin-irritating component. In such views, with no particular limitation, the amount of a component (particularly, nonvolatile component) other than the water-dispersed copolymer and the organic liquid component in the PSA composition (in turn, PSA) is, e.g. suitably approximately 10% by weight or less, preferably approximately 5% by weight or less, more preferably approximately 3% by weight or less, and may be 1.0% by weight or less. The art disclosed herein can be preferably performed using a composition containing such a limited amount of a component other than the water-dispersed copolymer and the organic liquid component.

### (Water-dispersed PSA Composition)

The PSA composition disclosed herein may be prepared using the water-dispersed copolymer and the organic liquid component as described above, thus allowing elimination of substantial use of an organic solvent or reduction in the amount of an organic solvent. This is significant in terms of independence from the use of an organic solvent, environmental friendliness, health of an operator, and the like. The PSA composition can be performed in the form of a water-dispersed PSA composition in which at least a part of a PSA-forming component is dispersed in an aqueous medium. Note that the term "water-dispersed" also encompasses a suspension form and an emulsion form. An aqueous medium refers to a substance in which a solvent contained in the medium is water or a mixed solvent containing water as a major component (an aqueous solvent). The concept of the water-dispersed PSA composition encompasses a substance referred to as an emulsified PSA composition. The PSA composition disclosed herein typically has an emulsion form containing the water-dispersed copolymer being dispersed in water.

### <PSA >

The PSA disclosed herein can be formed e.g., using the PSA composition by a heretofore known method. For example, the PSA composition can be applied onto a releasable surface (release side), allowed to dry, and thereby formed into a layered PSA (a PSA layer). In a PSA sheet having a support, for example, a method of forming a PSA layer by directly providing (typically, applying) the PSA composition onto the support followed by drying (a direct method) can be employed. Another method can also be employed in which the PSA composition is applied onto a releasable surface (release side), allowed to dry, and thereby formed into a PSA layer on the surface, followed by transfer of the PSA layer to a support (a transfer method). As the release side, for example, a surface of a release liner can be preferably used. The PSA layer disclosed herein is typically formed in a continuous form, but not limited to such a form, and may be formed, e.g., in a regular or random pattern such as dots or stripes.

The PSA composition can be applied, using a heretofore known coater, such as a gravure roll coater, a die coater, or a bar coater. Alternatively, the PSA composition can be applied by immersion, curtain coating, or another method. In view of promoting a cross-linking reaction, improving production efficiency, and the like, drying of the PSA composition is preferably performed under heating. The drying temperature can be, e.g., about 40°C to 150°C, and is preferably about 60°C to 130°C. After drying the PSA composition, aging may be performed for the purposes such as adjusting migration of components in a PSA layer, advancing a crosslinking reaction, and lessening possible strain in the PSA layer and the like.

As have been described so far, the specification provides a PSA containing a component that can be contained in the PSA composition disclosed herein. The PSA contains particularly a water-dispersed copolymer and an organic liquid component, and can further contain various optional components described above. The items describing the PSA composition are also applied to a PSA formed from the PSA composition except for a component volatile in formation of the PSA.

### <PSA sheet>

### (Configuration)

The PSA sheet disclosed herein is not particularly limited as long as it has a PSA (layer) formed from the PSA composition. The PSA sheet may be a PSA sheet with the PSA (layer) on one or each side of a non-releasable support, or a support-free PSA sheet in which the PSA (layer) is retained on a release liner (i.e., a PSA sheet free of a non-releasable support, typically a PSA sheet formed of a PSA layer). The concept of PSA sheet herein may encompass so-called PSA tape, PSA labels, PSA film, and the like. The PSA sheet disclosed herein can be in a roll form or in a flat sheet form. Alternatively, the PSA sheet may be processed into various shapes.

An exemplary configuration of the PSA sheet is depicted in Fig. 1. A PSA sheet 1 shown in Fig. 1 is formed as a one-sided PSA sheet including a PSA layer 10 having a first surface 10A that is a side to be attached to an adherend (skin), and a support 20 stacked on a second surface 10B of the PSA layer 10. The PSA layer 10 is in fixed contact with a first surface 20A of the support 20. Before use (before attaching to an adherend), the PSA sheet 1 may be in the form of an on-release liner PSA sheet 50 in which, for example, the adhesive side 10A is protected by a release liner 30 having a releasable surface (release side) at least on the PSA layer side, as shown in Fig. 1. Another form may be available in which a second side 20B (that is a surface opposite to the first side 20A and also referred to as a backside) of the support 20 is a release side, and the adhesive side 10A is protected by rolling or stacking so as to bring the adhesive side 10A into contact with the second side 20B of the support 20.

### (PSA Layer)

The thickness of the PSA layer is not particularly limited. The thickness of the PSA layer is, typically approximately 300 µm or less, suitably approximately 100 µm or less, preferably approximately 80 µm or less, and may be 60 µm or less. The PSA layer disclosed herein can be utilized as having such a limited thickness and providing good tight adhesiveness to skin. When having a limited thickness, the PSA layer can meet the requirement well for reduction in thickness or weight. The lower limit of the thickness of the PSA layer is not particularly limited, and in view of adhesive strength and conformability to skin as an adherend, it is, e.g., approximately 1 µm or more, suitably approximately 10 µm or more, preferably 20 µm or more, and may be, e.g., 30 µm or more.

### (Support)

In some embodiments, the PSA sheet may be in the form of a PSA sheet having a PSA layer on one or each side of a support. Examples of a support that can be used to support (back) the PSA layer include plastic films such as polyolefin (e.g., polyethylene, polypropylene, and ethylenepropylene copolymer) films, polyester (e.g., polyethylene terephthalate (PET)) films, vinyl chloride-based resin films, vinyl acetate-based resin films, polyimide-based resin films, polyamide-based resin films, fluororesin films, and cellophane; paper such as Japanese paper, kraft paper, glassine, woodfree paper, synthetic paper, and top-coated paper; woven or nonwoven fabrics composed of any of various types of fibrous substances, either singly or as a blend; rubber sheets made of natural rubber, butyl rubber, or the like; foam sheets made of polyurethane foam, polychloroprene rubber foam, or the like.; metal foils such as aluminum foils and copper foils; or a composite thereof. The plastic film may be of a non-stretched type or a stretched type (monoaxially stretched or biaxially stretched type). The support may be in a single layer form, or may be in a laminated form.

In some embodiments, as the support, nonwoven and woven fabrics, other porous films and the like are used. When using nonwoven and woven fabrics, their materials are not particularly limited, and one or two or more kinds of materials selected from natural fibers such as cotton, hemp, and wool; cellulose-based fibers such as rayon and acetate; polyamide fibers such as vinylon and nylon; polyolefin fibers such as polyethylene and polypropylene; polyester fibers such as polyethylene terephthalate; synthetic fibers such as polyurethane fibers; and the like can be used. Porous films include porous resin films formed of polypropylene, polyethylene, PET, polyurethane, or the like. In particular, nonwoven fabrics are preferred and nonwoven fabrics formed from synthetic fabrics such as polyester fabrics are more preferred. These support materials have suitable breathability and moisture permeability, can also have suitable elasticity/non-elasticity, and thus can be preferably employed for applications of use for attachment to skin.

The support may optionally contain various additives such as a filler (e.g., an inorganic filler and an organic filler), an anti-aging agent, an antioxidant, a UV absorber, an anti-static agent, a slip agent, a plasticizer, and a colorant (e.g., a pigment or a dye). A surface of the support (especially, the surface on which the PSA layer is formed) may be subjected to a known or conventional surface treatment such as corona discharge treatment, plasma treatment, and primer application. Such a surface treatment may be, e.g., a treatment for enhancing anchoring properties of the PSA layer to the support.

The thickness of the support can be appropriately selected corresponding to a purpose, and is typically approximately 10 µm or more, and may be, e.g., 20 µm or more or 30 µm or more. An increased thickness of the support is likely to lead to an improved strength of the support, the PSA sheet, or the like, providing good usability and easy handling. The upper limit of the thickness of the support is not particularly limited, and in some embodiments, it is, e.g., suitably approximately 1 mm or less, and may be approximately 500 µm or less, approximately 300 µm or less, or 100 µm or less. Limiting the thickness of the support tends to increase conformability to a surface structure (e.g., unevenness) of an adherend.

### (Release liner)

The release liner is not particularly limited and examples that can be used include a release liner in which a surface of a liner substrate such as resin film or paper is release-treated, and a release liner formed from a low-adhesive material such as fluoropolymer (e.g., polytetrafluoroethylene) or polyolefinic resin (e.g., polyethylene or polypropylene). In the release treatment, for example, a release agent such as a silicone-based or long-chain alkyl-based release agents can be used. In some embodiments, a release-treated resin film can be preferably employed as the release liner.

### (Adhesive Strength)

With no particular limitation, the adhesive strength of the PSA or PSA sheet in some embodiments is preferably approximately 0.5 N/10 mm or more, more preferably approximately 1.0 N/10 mm or more, even more preferably approximately 1.5 N/10 mm or more. With such an adhesive strength, the PSA or PSA sheet adheres well to skin and is less likely to shift or peel off while attached on the skin. In some embodiments, the adhesive strength is, e.g., suitably 3.0 N/10 mm or less, and may be 2.0 N/10 mm or less. With a limited adhesive strength to a predetermined value or less , the PSA sheet can provide a reduced peel load to skin while peeled off from the skin, and help peeling from the skin with no damage such as exfoliation of keratin layers. The adhesive strength is a 180° peel strength measured using a Bakelite plate as an adherend. The adhesive strength to a Bakelite plate (to-Bakelite-plate adhesive strength) can be measured, specifically by the method described later in the examples.

### (Total Thickness)

The total thickness of the PSA sheet (including a PSA layer and support, but not a release liner) disclosed herein is not particularly limited. In some embodiments, the total thickness of the PSA sheet can be, e.g., approximately 1 mm or less, and may be, in view of handling or the like, approximately 500 µm or less, approximately 300 µm or less, or approximately 100 µm or less. With a limited thickness, the PSA sheet can provide a product with less weight and thickness. The lower limit of the PSA sheet is not particularly limited, and in view of adhesive properties (e.g., adhesive strength), handling, and the like, it is, e.g., suitably approximately 10 µm or more, preferably approximately 20 µm or more, more preferably approximately 30 µm or more, and may be approximately 50 µm or more, in some embodiments.

### <Applications>

The PSA composition, PSA and PSA sheet disclosed herein are preferred for medical applications for attachment to skin by taking advantage of features of less irritation to skin in peeling from the skin, good water resistance, and further, a capability of preventing pollution of skin by a PSA component. For example, they can be used as various medical PSAs and medical PSA sheets, for the purpose of protection of an affected area of skin, transdermal absorption of a drug, and fixation of gauze, a tube, or the like to skin. With no particular limitation, the medical PSA and medical PSA sheet disclosed herein can be preferably used in the forms of, e.g., adhesive bandages, surgical tapes, first-aid adhesive tapes, large adhesive tapes, dressing materials, and cataplasm materials.

### [Examples]

Several examples related to the present invention will be described below, but are not intended to limit the present invention to the examples. In the description below, "parts" and "%" are indicated on a weight basis unless otherwise specified.

### <Preparation of Water-dispersed Acrylic Copolymer>

### (Preparation Example A1)

To a flask equipped with a reflux condenser, a thermometer, and a dropping funnel were added 85 g of water and 10 g of a reactive emulsifer (product name: "AQUALON KH-1025", manufactured by DKS Co., Ltd., solid concentration: 25%). Meanwhile, a mixture was prepared by adding 350 g of 2-ethylhexylacrylate (hereinafter abbreviated as 2EHA), 7.3 g of acrylic acid (hereinafter abbreviated as, AA), 3.7 g of *N*-vinylpyrrolidone (hereinafter abbreviated as, NVP), and 3.7 g of methacrylic acid (hereinafter abbreviated as, MAA) as monomers, and further adding 0.18 g of a silane-based monomer (product name: "KBM-503", manufactured by Shin-Etsu Chemical Co., Ltd., 3-methacryloxypropyltrimethoxysilane), 0.18 g of t-lauryl mercaptan as a chain transfer agent, 25 g of a reactive emulsifier (product name: "AQUALON KH-1025"), and 140 g of water, and emulsified with a high-pressure homogenizer to provide an emulsion (an emulsion of a monomer mixture). Forty grams of the emulsion was added to the flask and subjected to nitrogen replacement for 1 hour. Then, after heating to 60°C, 0.1 g of a water-soluble polymerization initiator (product name: "VA-057", manufactured by FUJIFILM Wako Pure Chemical Corporation) was added to start polymerization. After 1 hour, 0.1 g of the water-soluble polymerization initiator (product name: "VA-057") was further added, and a predetermined amount of the emulsion thus prepared above was dripped for 3 hours to perform an emulsion polymerization reaction. Subsequently, the reaction product was heated to 70°C and further kept for 3 hours. After cooling to room temperature, the water dispersion that underwent the polymerization reaction was adjusted to pH 5.0 by adding 10% ammonia water, and filtered with a Teflon^{®} mesh to remove aggregation, thereby providing a water dispersion of an acrylic copolymer A1(solid concentration: 54%).

### (Preparation Example A2)

A composition of an emulsion of a monomer mixture was changed, except for water, to 304 g of 2EHA, 6.8 g of AA, 27.1 g of methyl methacrylate (hereinafter abbreviated as, MMA), 0.07 g of the silane-based monomer, 0.04 g of t-lauryl mercaptan, and 1.6 g of an unreactive emulsifier (product name: "HITENOL LA16", manufactured by DKS Co., Ltd., polyoxyethylene lauryl alkyl ether ammonium sulfate). Other components was formulated with the same formulation as in Preparation Example A1, thereby providing a water dispersion of an acrylic copolymer A2.

### (Example 1)

Sorbitan monolaurate (product name : "RHEODOL SP-L10", manufactured by Kao Corporation, having a liquid form at normal temperature, the number of hydroxy groups per molecule: 3, HLB: 8.6) as an organic liquid component was added to the water dispersion of an acrylic copolymer A1 so as to provide 30 parts relative to 100 parts of a solid content of the water dispersion A1, stirred under conditions at 2,000 revolutions/min for 10 minutes with the product named as "Awatori Rentarou" manufactured by Thinky Corporation, and then deformed under conditions at 2,200 revolutions/min for 2 minutes to provide a PSA composition.

The PSA composition was applied on a release-treated side of a release liner including a PET film release-treated with a silicone-based material on one side and having a thickness of 38 µm (product name: "MRF38", manufactured by Mitsubishi Chemical Group Corporation), and dried at 130°C for 3 minutes, thereby forming a PSA layer with a thickness of 50 µm. An exposed adhesive side of the PSA layer was protected by covering with a release liner including a PET film release-treated with a silicone-based material on one side and having a thickness of 38 µm (manufactured by Mitsubishi Chemical Group Corporation, product name: "MRE38"). In this manner, a PSA sheet including a PSA layer having each side protected by a release liner was prepared.

### (Examples 2 to 6 and Comparative Examples 1 to 5)

A PSA sheet according to each example was prepared in the same manner as in Example 1 except for changing a component used and the amount as shown in Table 1.

In Table 1, the product named "RHEODOL SP-O10V" (manufactured by Kao Corporation, having a liquid form at normal temperature, the number of hydroxy groups per molecule: 3, HLB: 4.3) was used as sorbitan monooleate; the product named as "Tween 20" (manufactured by Tokyo Chemical Co., Ltd., having a liquid form at normal temperature, the number of hydroxy groups per molecule: 3, HLB: 16.7) was used as polyoxyethylenesorbitan monolaurate; the product named as "Tween 80" (manufactured by Tokyo Chemical Co., Ltd., having a liquid form at normal temperature, the number of hydroxy groups per molecule: 3, HLB: 10.0) was used as polyoxyethylenesorbitan monooleate; the product named as "RHEODOL SP-O30V" (manufactured by Kao Corporation, having a liquid form at normal temperature, the number of hydroxy groups per molecule: 1, HLB: 1.8) was used as sorbitan trioleate; the product named as "RHEODOL SP-S30V" (manufactured by Kao Corporation, the number of hydroxy groups per molecule: 1, HLB: 2.1) as sorbitan tristearate; and dimer acid manufactured by Tokyo Chemical Co., Ltd. (HLB 14) was used as dimer acid.

### <Evaluation>

### [180° Peel Strength (Adhesive Strength)]

In a PSA sheet according to each example, a release liner covering a first adhesive side was peeled, and a PET film with a thickness of 25 µm (product name: "LUMIRROR S10", manufactured by Toray Industries, Inc.) was attached for backing, thereby providing a measurement sample. The measurement sample is cut into a size of 5 cm × 1 cm, and then under an environment at room temperature (23°C), a second adhesive side was exposed, attached onto a Bakelite plate as an adherend, and pressure-bonded with a 2 kg roller moved back and forth once. Under the same environment, the load at peeling a PSA sheet from the adherend was measured under conditions at a peel angle of 180° and a tensile speed of 300 mm/min using a tensile tester (manufactured be Shimadzu Corporation, product name: "Autograph AG-Xplus HS 6000 mm/min high-speed model (AG-50NX plus)"), and the peel strength to the Bakelite plate (to-Bakelite plate adhesive strength) [N/10 mm] was calculated. As the Bakelite plate, a phenol laminate (paper phenol) manufactured by Standard Testpiece Co., Ltd. was used.

In addition, for a sample that generates anchor failure from a backing PET film in measuring a peel strength, assessment was performed using a sample obtained by directly applying a PSA composition onto a PET film with a thickness of 25 µm (product name: "LUMIRROR S10", manufactured by Toray Industries, Inc.) to form a PSA layer with a thickness of 50 µm.

### [To-skin adhesive strength ]

A PSA sheet according to each example is cut into a size of 5 cm × 5 cm, followed by peeling of a release liner covering a first adhesive side, and attached onto an edge of a polyester spunlace non-woven fabric (product name: "SONTARA #8010", manufactured by Nissei Co., Ltd., area weight: 44 g/m²) cut into a size of 5 cm × 25 cm, thereby a measurement sample. A release liner covering a second adhesive side was peeled from the measurement sample, and an exposed adhesive side of the measurement sample is attached onto an inner arm of a subject and pressed gently for 10 seconds with an opposed hand under an environment at room temperature. Subsequently, under the same environment, the load at peeling of the PSA sheet from the arm was measured under conditions at a peel angle of 180° and a tensile speed of 300 mm/min using the tensile tester, and the peel strength (to skin adhesive strength) [N/50 mm] was calculated.

In addition, for a sample that generates anchor failure from a non-woven fabric in measuring a peel strength, assessment was performed using a measuring sample derived by applying a PSA composition onto a release liner, then attaching it onto the non-woven fabric before drying, and drying at 130°C for 3 minutes.

### [Skin Irritancy]

A measurement sample was prepared by the method described above for to-skin adhesive strength, using a PSA sheet and polyester spunlace non-woven fabric. An exposed adhesive side of the measurement sample was attached onto an inner arm of a subject, and pain at tearing off of the measurement sample from the inner arm was assessed after 2 hours, and judged in accordance with the following criteria.
AA: Feel no pain at all.
A: Feel discomfort but little pain.
B: Feel skin pulling but a limited pain.
C: Suffering.
Judgement was made as pass if an evaluation result was B or above, and as fail if the result was C.

### [Water resistance ]

A measurement sample was prepared by backing a PET film (product name: "LUMIRROR S10", manufactured by Toray Industries, Inc., thickness: 25 µm) with a PSA sheet, or directly applying a PSA composition on the PET film. The measurement sample was cut into a size of 2 cm × 6 cm, and attached onto a slide glass (product number: "S1111", manufactured by Matsunami Glass Ind., Ltd.). The measurement sample was put in a plastic container, then water was poured in the container up to complete immersion of the measurement sample, followed by treatment with an ultrasonic washer for 1 hour, and the degree of permeation of water into a PSA layer was visually observed and judged in accordance with the following criteria.
A: Observe no change in a PSA layer.
B: Observe cloudiness only around (on the outer periphery of) a PSA layer.
C: Observe cloudiness into a PSA layer, or release of the PSA layer from a PET film.

### [Skin Pollution]

A PSA sheet was attached onto a polyester spunlace non-woven fabric (product name: "SONTARA #8010", manufactured by Nissei Co., Ltd., area weight: 44 g/m²) and cut into a size of 2 cm × 5 cm, thereby providing a measurement sample. An adhesive side of the measurement sample was attached onto the outside of a vicinity of the right wrist of a subject, and quickly peeled from the skin after 1 hour. Presence or absence of pollution of the skin by a PSA after peeling of the measurement sample was visually observed, and judged in accordance with the following criteria.
A: Observe no pollution by a PSA.
B: Observe a slight residue, but no tuck occurred even at a touch.
C: Observe a residue, and a tuck occurred at a touch.

Judgement was made as pass if an evaluation result was A, and as fail if the result was B or below.

Table 1 shows a summary and evaluation results of each example.

### [Table 1]

**Table 1**

| | Water-dispersed copolymer | | Added component | | | Adhesive strength | | Skin irritancy | Water resistance | Skin pollution |
|---|---|---|---|---|---|---|---|---|---|---|
| | Component | Parts | Component | HLB | Parts | To-Bakelite-plate [N/10 mm] | To-skin [N/50 mm] | | | |
| Example 1 | A1 | 100 | sorbitan monolaurate | 8.6 | 30 | 1.5 | 1.1 | AA | A | A |
| Example 2 | A1 | 100 | sorbitan monooleate | 4.3 | 30 | 1.8 | 1.7 | B | A | A |
| Example 3 | A1 | 100 | polyoxyethylenesorbitan monolaurate | 16.7 | 30 | 1.1 | 0.8 | AA | B | A |
| Example 4 | A1 | 100 | polyoxyethylenesorbitan monooleate | 10.0 | 30 | 1.3 | 0.9 | AA | A | A |
| Example 5 | A1 | 100 | sorbitan monolaurate | 8.6 | 50 | 0.8 | 0.4 | AA | B | A |
| Example 6 | A1 | 100 | sorbitan monooleate | 4.3 | 50 | 1.6 | 1.5 | A | A | A |
| Comparative Example 1 | A1 | 100 | sorbitan trioleate | 1.8 | 30 | 4.2 | 4.7 | C | A | B |
| Comparative Example 2 | A1 | 100 | sorbitan tristearate | 2.1 | 30 | 3.9 | 3.8 | C | A | B |
| Comparative Example 3 | A1 | 100 | dimer acid | 14 | 30 | 4.5 | 3.6 | C | C | C |
| Comparative Example 4 | A2 | 100 | sorbitan monolaurate | 8.6 | 30 | 1.4 | 1.8 | B | C | A |
| Comparative Example 5 | A1 | 100 | sorbitan trioleate | 1.8 | 50 | 3.8 | 3.3 | C | A | C |

As shown in Table 1, Examples 1 to 6, which employed a PSA containing a water-dispersed copolymer obtained by copolymerizing a monomer mixture containing an alkyl (meth)acrylate, a carboxy group-containing monomer, an N-containing monomer, and a silane-based monomer, and an organic liquid component having more than one hydroxy group in a molecule, were judged as pass in evaluation results for skin irritancy, had good water resistance, and provided no skin pollution observed. On the other hand, Comparative Examples 1 to 3 and 5, which employed an organic component having one hydroxy group, were judged as fail in evaluation results for skin irritancy, and also exhibited poorer evaluation results for skin pollution. In Example 1 to 6, an organic liquid component containing more than one hydroxy group and having a suitable hydrophilicity may be well arranged on a surface of a polymeric particle in a PSA, and plasticizes the PSA wholly and suitably as well as forms a weak boundary layer (WBL) on a surface of a PSA layer, thereby reducing irritation to skin in peeling. This consideration is also supported by measurement results of adhesive strength of Examples 1 to 6. It should be noted that Examples 1 to 6 exhibited reduced adhesive strength as compared to Comparative Examples, but provided improved tight adhesiveness to skin by suitable plasticization as described above, resulting in good tight adhesiveness to skin. In contrast, in Comparative Examples 1 to 3, an organic component having one or less hydroxy group in a molecule presumably entered the interior of a polymeric particle in a PSA, causing a peel-off mode similar to cohesive failure; this may be reflected in evaluation results for skin irritancy and skin pollution, and measurement results for adhesive strength. As can be confirmed from the results of Comparative Example 3, the number of hydroxy groups rather than HLB of an organic liquid component significantly affects results of skin irritancy, water resistance, and skin pollution. In the use of sorbitan trioleate as an organic liquid component, comparison between Comparative Examples 1 and 5 shows that increase in the amount of sorbitan trioleate failed to improve skin irritancy, and instead, that the increase tended to make skin pollution worse. In Comparative Example 4, which employs a water-dispersed copolymer prepared with no N-containing monomer, the use of an organic liquid component led to pass in evaluation results for skin irritancy, but failed to provide good water resistance. Comparative Example 4 has a PSA composition corresponding to that of Example 4 in Patent Document 2, and the results of Comparative Example 4 suggests that PSAs of Examples 1 to 6 have more excellent water resistance than a PSA described in Patent Document 2. The water-dispersed copolymer A2 used in Comparative Example 4 had consequently lower adhesive strength than the water-dispersed copolymer A1 used in Example 1, but had, after addition of an organic liquid component (sorbitan monolaurate), higher adhesive strength than that in Example 1, and also exhibited observable difference in the degree of skin irritancy. These results may show that combined use of a water-dispersed copolymer synthesized using an N-containing monomer in addition to an alkyl (meth)acrylate, a carboxy group-containing monomer, and a silane-based monomer, with an organic liquid component having more than one hydroxy group can provide a more excellent effect of preventing skin irritation.

These results suggest that a PSA containing a water-dispersed copolymer obtained by copolymerizing a monomer mixture containing an alkyl (meth)acrylate, a carboxy group-containing monomer, an N-containing monomer, and a silane-based monomer, and an organic liquid component having more than one hydroxy group in a molecule can have less irritation to skin in peeling from the skin, good water resistance, and further provide excellent prevention of pollution of skin by a PSA component.

Although specific embodiments of the present invention have been described in detail so far, these are merely for illustrations and do not limit the scope of the claims. The art recited in the claims includes various modifications and changes made to the specific embodiments illustrated above.

### [Reference Signs List]

1 PSA sheet
10 PSA layer
10A first surface (adhesive side)
10B second surface
20 support
20A first side
20B second side (backside)
30 release liner
50 on-release liner PSA sheet

## Claims

1. A medical pressure-sensitive adhesive comprising:
a water-dispersed copolymer obtained by copolymerizing a monomer mixture comprising an alkyl (meth)acrylate, a carboxy group-containing monomer, a nitrogen-atom-containing monomer, and a silane-based monomer, and
an organic liquid component having more than one hydroxy group in a molecule.

2. The medical pressure-sensitive adhesive according to claim 1, wherein the nitrogen-atom-containing monomer is contained in an amount of 0.1 to 10 parts by mass in 100 parts by mass of the monomer mixture.

3. The medical pressure-sensitive adhesive according to claim 1 or 2, wherein the nitrogen-atom-containing monomer has a structure in which a nitrogen atom is bound to no hydrogen atoms.

4. The medical pressure-sensitive adhesive according to claim 1 or 2, wherein the nitrogen-atom-containing monomer has a cyclic structure comprising a nitrogen atom in a ring.

5. The medical pressure-sensitive adhesive according to claim 1 or 2, wherein the carboxy group-containing monomer is contained in an amount of 0.1 to 10 parts by mass in 100 parts by mass of the monomer mixture.

6. The medical pressure-sensitive adhesive according to claim 1 or 2, wherein the organic liquid component has a hydrophile-lipophile balance (HLB) of 3 to 16.

7. The medical pressure-sensitive adhesive according to claim 1 or 2, wherein the organic liquid component is a fatty acid ester derivative of polyhydric alcohol.

8. The medical pressure-sensitive adhesive according to claim 7, wherein a fatty acid forming the fatty acid ester derivative of polyhydric alcohol is a fatty acid having 8 to 18 carbons.

9. The medical pressure-sensitive adhesive according to claim 1 or 2, wherein the organic liquid component is contained in an amount of 1 to 60 parts by mass relative to 100 parts by mass of the water-dispersed copolymer.

10. A medical pressure-sensitive adhesive sheet having the medical pressure-sensitive adhesive according to claim 1 or 2 on at least one side of a support.
